Europäisches Patentamt

European Patent Office

Office européen des brevets

(19)

(11) Veröffentlichungsnummer: **0 304 763**

**A1**

# (12) EUROPÄISCHE PATENTANMELDUNG

(21) Anmeldenummer: 88113245.0

(22) Anmeldetag: 16.08.88

(51) Int. Cl.⁴: **C07C 59/125 , C07C 51/235**

(30) Priorität: 24.08.87 DE 3728222

(43) Veröffentlichungstag der Anmeldung:
**01.03.89 Patentblatt 89/09**

(84) Benannte Vertragsstaaten:
**AT BE CH DE ES FR GB GR IT LI LU NL SE**

(71) Anmelder: **Henkel Kommanditgesellschaft auf Aktien**
**Postfach 1100 Henkelstrasse 67**
**D-4000 Düsseldorf-Holthausen(DE)**

(72) Erfinder: **Tran-Vinh, Loc, Dr.**
**Eichenstrasse 145**
**D-4010 Hilden(DE)**
Erfinder: **Göbel, Gerd, Dr.**
**Lindenstrasse 10**
**D-4006 Erkrath(DE)**
Erfinder: **Carduck, Franz-Josef, Dr.**
**Landstrasse 18**
**D-5657 Haan(DE)**
Erfinder: **Herbst, Sigurd, Dr.**
**Auf der Driesch 14**
**D-5000 Köln 91(DE)**

(54) **Verfahren zur Herstellung von Ethercarbonsäuren.**

(57) Bei Verfahren zur Herstellung von Ethercarbonsäuren der allgemeinen Formel (I)

$$R-(O-C_mH_{2m})_n-OCH_2COOH \qquad (I)$$

durch Oxidation von Polyalkoxyalkoholen der allgemeinen Formel (II)

$$R-(O-C_mH_{2m})_n-OCH_2CH_2OH \qquad (II)$$

in flüssiger Phase mit Sauerstoff bzw. Sauerstoff enthaltenden Gasen in Gegenwart von Edelmetallkatalysatoren erreicht man erheblich kürzere Reaktionszeiten, wenn man der flüssigen Phase vor Beginn der Umsetzung 0,0001 bis 1 Mol einer Verbindung der Formel (I), bezogen auf eingesetzte Verbindungen der Formel (II), zusetzt.

EP 0 304 763 A1

## Verfahren zur Herstellung von Ethercarbonsäuren

Die Erfindung betrifft ein Verfahren zur Herstellung von Ethercarbonsäuren der allgemeinen Formel (I)

$$R\text{-}(O\text{-}C_mH_{2m})_n\text{-}OCH_2COOH \qquad (I)$$

in der
R eine lineare oder verzweigte Alkylgruppe mit 1 bis 20 Kohlenstoffatomen, eine Alkylarylgruppe oder eine Arylgruppe,
m die Zahl die 2 und/oder 3 und
n eine Zahl von 0 bis 20
bedeuten,
durch Oxidation von Polyalkoxyalkoholen bzw. Fettalkoholalkoxylaten der allgemeinen Formel (II)

$$R\text{-}(O\text{-}C_mH_{2m})_n\text{-}OCH_2CH_2OH \qquad (II)$$

in der R, m und n wie oben definiert sind, in flüssiger, insbesondere wäßrig-alkalischer Phase mit Sauerstoff bzw. Sauerstoff enthaltenden Gasen in Gegenwart von wirksamen Mengen eines Katalysators aus Edelmetallen der Nebengruppe VIII des Periodensystems bzw. Verbindungen dieser Edelmetalle sowie gegebenenfalls eines Katalysator/Aktivators. Ethercarbonsäuren der Formel (I) sind wichtige Zwischenprodukte für die Herstellung von Tensiden, die beispielsweise in kosmetischen Produkten eingesetzt werden.

Als Beispiele für die Gruppe R der allgemeinen Formel (I) bzw. (II) sind Methyl- oder Ethylgruppen zu nennen, weiterhin lineare oder verzweigte Alkylgruppen wie Propyl-, Butyl-, Pentyl-, Hexyl-, Heptyl-, Octyl-, Nonyl- und Decylgruppen. Besonders häufig werden Ethercarbonsäuren hergestellt, deren Rest R von $C_{12}$-$C_{18}$-Fettalkoholen bzw. technischen Gemischen derselben pflanzlichen, tierischen und/oder seetierischen Ursprungs abgeleitet sind. Als typisches Beispiel für Alkylarylgruppen ist die Alkylphenylgruppe zu nennen, die mit 1 bis 3 Alkylgruppen alkylsubstituiert sein kann, wobei die Summe der Kohlenstoffatome in den Alkylsubstituenten 1 bis 16 betragen kann. Ein wichtiger Vertreter für einen Arylsubstituenten ist die Phenylgruppe.

Die in den Verbindungen (I) bzw. (II) auftretende Funktion der Formel

$$\text{-}(O\text{-}C_mH_{2m})_n\text{-}$$

bildet sich bei der Umsetzung der Alkohole ROH mit Ethylenoxid und/oder Propylenoxid, wobei im Falle der Ethylenoxid/Propylenoxid-Addukte die Propylenglykolreste sich in der Alkoxylatkette in random- oder block-Verteilung befinden können.

Ein Verfahren der obengenannten Art zur Herstellung von Ethercarbonsäuren der allgemeinen Formel (I) unter Verwendung von Katalysator/Aktivatoren ist aus der DE-A 31 35 946 bekannt. Ähnliche Verfahren, die ohne Katalysator/Aktivatoren arbeiten, sind in der US-B 3 342 858, DE-A 28 16 127 und DE-A 34 46 561 beschrieben. Die Oxidation mit Platin/Palladium-Katalysatoren ist aus der EP-A 00 73 545, die Oxidation in neutralen Medien aus der DE-A 29 36 123 bekannt. Andere Veröffentlichungen beschreiben die katalytische Oxidation mit Wasserstoffperoxid (EP-A 00 39 111) und mit t-Butylperoxid (EP-A 00 18 681).

Die vorliegende Erfindung beruht auf der überraschenden Erkenntnis, daß man bei der Herstellung von Ethercarbonsäuren der allgemeinen Formel (I) eine erhebliche Verkürzung der Reaktionszeiten sowohl bei kontinuierlicher als auch bei diskontinuierlicher Verfahrensführung erreicht, wenn man dem Reaktionsgemisch vor Beginn der Umsetzung eine wirksame Menge an Ethercarbonsäuren der allgemeinen Formel (I) zusetzt.

Demgemäß ist das Verfahren der Erfindung dadurch gekennzeichnet, daß man der flüssigen Phase vor Beginn der Umsetzung 0,0001 bis 1 Mol einer Verbindung gemäß Formel (I), pro Mol Polyalkoxylat bzw. Fettalkoholalkoxylat der Formel (II), zusetzt. Das Molverhältnis von Produkt zu Edukt zu Reaktionsbeginn kann durch Optimierung ermittelt werden, so daß das Verfahren, beispielsweise bei kontinuierlicher Verfahrensweise, wirtschaftlich ist, indem die mittlere Verweilzeit in der gesamten Anlage auf ein Minimum gesenkt wird. Hinsichtlich der Wirtschaftlichkeit des Verfahrens ist ein Zusatz in der Größenordnung von 0,1 bis 0,4 Mol Ethercarbonsäure pro Mol Polyalkoxylat bzw. Fettalkoholalkoxylat bevorzugt.

Bevorzugt setzt man dem Reaktionsgemisch vor Beginn der Umsetzung eine Verbindung der Formel (I) zu, die mit dem bei der Reaktion zu erwartenden Reaktionsprodukt identisch ist. Man kann jedoch auch andere Ethercarbonsäuren der Formel (I) zusetzen, z.B. wenn diese billiger oder leichter zugänglich als die erst mit dem Verfahren der Erfindung zu erzeugenden Ethercarbonsäuren sind.

Das Verfahren der Erfindung bietet darüber hinaus noch den zusätzlichen Vorteil, daß z.B. bei der Oxidation linearer gesättigter $C_{12}$-$C_{14}$-Fettalkoholpolyglykolethern mit durchschnittlich 4 Ethylenoxid-Einheiten diese oberhalb des Trübungspunktes des Ethoxylats durchgeführt wird und das Substrat sich durch die zugesetzte Ethercarbonsäure emulgieren läßt, wobei man unter den Bedingungen einer micellaren Katalyse arbeitet.

Vorzugsweise wird das Verfahren der Erfindung in wäßrig-alkalischen Medien durchgeführt; man kann jedoch auch in Anwesenheit von inerten organischen Lösemitteln arbeiten. Die Reaktionstemperatur liegt im Bereich zwischen 0°C bis zum Siedepunkt des Reaktionsgemisches, vorzugsweise bei 40 bis 100°C.

Der für das Verfahren der Erfindung erforderliche Betriebsdruck ist abhängig von dem Partialdruck des Sauerstoffs in dem verwendeten Gas. Der Druckbereich kann zwischen 0,5 bis 40 bar liegen.

Die Einstellung des pH-Wertes erfolgt durch Zugabe von Alkalien, vorzugsweise der Hydroxide und/oder Carbonate des Natriums und/oder Kaliums. Im allgemeinen setzt man etwa 1 bis 1,5 Mol Alkali pro Mol Polyalkoxyalkohol ein. Die Alkalien können zu Reaktionsbeginn auf einmal oder erst im Reaktionsverlauf hinzugefügt werden.

Als Katalysatoren eignen sich insbesondere Platin und/oder Palladium in metallischer Form oder in Form von katalytisch wirksamen Verbindungen derselben. Die Katalysatoren können auch auf festen Trägern wie Aktivkohle, Silikaten, Aluminiumoxid und dergleichen aufgebracht sein. Der Edelmetallgehalt der Trägerkatalysatoren kann in weiten Grenzen schwanken und beträgt vorzugsweise 0,1 bis 20 Gew.-%, bezogen auf das Trägermaterial.

Die Aktivität und Selektivität der Katalysatoren können durch Zusatz von Aktivatoren, z.B. von Blei, Wismut sowie gegebenenfalls zusätzlich Cadmium bei Platinkatalysatoren oder Wismut bei Palladiumkatalysatoren gesteigert werden, vgl. DE-A 31 35 946.

Das Verfahren der Erfindung kann in sämtlichen Apparaturen, die sich für die Durchführung von Flüssigphasen-Reaktionen mit oder ohne Anwendung von Überdruck eignen, durchgeführt werden, z.B. in einem Rührkessel oder in einer Blasensäule mit suspendiertem Katalysator oder auch in einem Rieselbettreaktor mit Katalysatorpellets.

Das gemäß dem Verfahren der Erfindung erhaltene Reaktionsprodukt wird in Form einer wäßrigen Lösung seiner Alkalisalze erhalten, welche als solche, z.B. als Tensid, eingesetzt werden kann. Man kann jedoch auch die freien Ethercarbonsäuren durch Ansäuern und gegebenenfalls Extrahieren auf an sich bekannte Weise freisetzen und isolieren.

Das Verfahren der Erfindung wird im folgenden anhand von bevorzugten Ausführungsbeispielen näher erläutert.

Beispiel 1

In einen mit Drehrührer, Innenthermometer und Gaszuleitung versehenen, über einen Heizmantel temperierbaren 3 1-Autoklaven aus Hastelloy B wurden 500 g destilliertes Wasser, 12,97 g Aktivkohle mit 5 Gew.-% Palladiumgehalt, 0,245 g Bi-$(NO_3)_3$ als Aktivator, 20,76 g Ätznatron und 157,4 g eines linearen, gesättigten $C_{12}$-$C_{14}$-Fettalkoholpolyglykolethers (70 bis 75 % $C_{12}$, 24 bis 30 % $C_{14}$) mit durchschnittlich 4 Ethylenoxid-Einheiten gegeben. Weiterhin wurden 18,26 g Ethercarbonsäure aus dem vorstehend genannten Fettalkoholpolyglykolether (0,1 Mol, bezogen auf den Fettalkoholpolyglykolether) zugesetzt.

Nach dem Anstellen des Rührers wurde das Reaktionsgemisch auf 90°C erwärmt. Bei dieser Temperatur wurd Syntheseluft (21 Vol.-% $O_2$) bei 20 bar durch den Reaktor mit einem Volumenstrom von 30 N1 Luft pro Stunde geleitet. Der Reaktionsverlauf wurde am Reaktorausgang mit einem Sauerstoffanalysator verfolgt.

Nach dem Abfiltrieren des Katalysators und der Bismutverbindung wurde das Filtrat mit verdünnter Schwefelsäure angesäuert und zur Ausbeutebestimmung mehrfach mit Ether ausgeschüttelt. Von den vereinigten Etherextrakten wurde der Ether nach dem Trocknen über Natriumsulfat abgezogen. Der Rückstand wurde gewogen; seine Säurezahl wurde bestimmt.

Durch Vergleich der experimentell bestimmten Säurezahl des erhaltenen Produktes mit dem zu erwartenden theoretischen Wert von 132,9 ließ sich ein Umsatz von 100 % ermitteln.

Vergleichsbeispiel I.

Das Beispiel 1 wurde wiederholt, jedoch ohne Zusatz der Ethercarbonsäure aus dem eingesetzten Fettalkoholpolyglykolether. Ein vollständiger Umsatz (100 %) wurde erst nach 7,5 Stunden Reaktionszeit erreicht. Somit war die Reaktionszeit ohne Zusatz an Ethercarbonsäure um 30 % höher als beim Beispiel 1.

Beispiel 2

In einen Autoklaven gemäß Beispiel 1 wurden 500 g destilliertes Wasser, 20 g Aktivkohle mit 5 Gew.-% Platingehalt, 7,96 g Ätznatron und 100 g eines linearen, gesättigten $C_{12}$-$C_{18}$-Fettalkoholpolyglykolethers mit durchschnittlich 10 Ethylenoxid-Einheiten gegeben; weiterhin wurden 18,26 g Ethercarbonsäure aus dem vorstehend genannten Fettalkoholpolyglykolether zugesetzt (0,1 Mol, bezogen auf eingesetzten Fettalkoholpolyglykolether).

Bei einer Reaktionstemperatur von 90°C wurde Syntheseluft (mit 21 Vol.-% $O_2$) bei 20 bar Reaktionsdruck durch den Reaktor mit einem Volumenstrom von 30 N1 Luft pro Stunde geleitet. Der Versuch wurde nach 6,25 Stunden abgebrochen, nachdem kein Sauerstoffverbrauch mehr feststellbar war; der Umsatz wurde in der in Beispiel 1 beschriebenen Weise mit 100 % ermittelt (theoretische Säurezahl = 84,9).

Vergleichsbeispiel II.

Es wurde der Versuch des Beispiels 2 wiederholt, jedoch ohne Zusatz an Ethercarbonsäure. Der Versuch wurde nach 6,25 Stunden abgebrochen. Es wurde ein Umsatz von 81,3 % festgestellt.

**Ansprüche**

1. Verfahren zur Herstellung von Ethercarbonsäuren der allgemeinen Formel (I)

$$R\text{-}(O\text{-}C_mH_{2m})_n\text{-}OCH_2COOH \qquad (I)$$

in der
R eine lineare oder verzweigte Alkylgruppe mit 1 bis 20 Kohlenstoffatomen, eine Alkylarylgruppe oder eine Arylgruppe,
m die Zahl 2 und/oder 3 und
n eine Zahl von 0 bis 20
bedeuten,
durch Oxidation von Polyalkoxyalkoholen bzw. Fettalkoholalkoxylaten der allgemeinen Formel (II)

$$R\text{-}(O\text{-}C_mH_{2m})_n\text{-}OCH_2CH_2OH \qquad (II)$$

in der R, m und n wie oben definiert sind, in flüssiger, insbesondere wäßrig-alkalischer Phase mit Sauerstoff bzw. Sauerstoff enthaltenden Gasen in Gegenwart von wirksamen Mengen eines Katalysators aus Edelmetallen der Nebengruppe VIII des Periodensystems bzw. Verbindungen dieser Metalle sowie ggf. eines Katalysator/Aktivators, dadurch gekennzeichnet, daß man der flüssigen Phase vor Beginn der Umsetzung 0,0001 bis 1 Mol einer Verbindung gemäß Formel (I), bezogen auf Polyalkoxylate bzw. Fettalkoholalkoxylate der Formel (II), zusetzt.

2. Verfahren nach Anspruch 1, dadurch gekennzeichnet, daß man 0,1 bis 0,4 Mol der Verbindung gemäß Formel (I), bezogen auf Polyalkoxylate bzw. Fettalkoholalkoxylate der Formel (II), zusetzt.

3. Verfahren nach Anspruch 1 oder 2, dadurch gekennzeichnet, daß man eine vor Beginn der Umsetzung zuzusetzende Verbindung der Formel (I) verwendet, die mit dem Reaktionsprodukt identisch ist.

Europäisches
Patentamt

# EUROPÄISCHER RECHERCHENBERICHT

| | EINSCHLÄGIGE DOKUMENTE | | EP 88113245.0 |
|---|---|---|---|
| **Kategorie** | **Kennzeichnung des Dokuments mit Angabe, soweit erforderlich, der maßgeblichen Teile** | **Betrifft Anspruch** | **KLASSIFIKATION DER ANMELDUNG (Int. Cl.4)** |
| D,A | DE - A1 - 3 185 946 (BAYER AG)<br><br>* Ansprüche *<br><br>-- | 1 | C 07 C 59/125<br>C 07 C 51/235 |
| D,A | DE - A1 - 3 446 561 (AGIP)<br><br>* Ansprüche *<br><br>---- | 1 | |

**RECHERCHIERTE SACHGEBIETE (Int. Cl.4)**

C 07 C 51/00

C 07 C 59/00

Der vorliegende Recherchenbericht wurde für alle Patentansprüche erstellt.

| Recherchenort | Abschlußdatum der Recherche | Prüfer |
|---|---|---|
| WIEN | 11-11-1988 | HOFBAUER |